**Europäisches Patentamt**

**European Patent Office**

**Office européen des brevets**

(19)

(11) **EP 0 792 121 B1**

(12) **EUROPÄISCHE PATENTSCHRIFT**

(45) Veröffentlichungstag und Bekanntmachung des Hinweises auf die Patenterteilung:
**20.05.1998 Patentblatt 1998/21**

(21) Anmeldenummer: **95937759.9**

(22) Anmeldetag: **14.11.1995**

(51) Int. Cl.$^6$: **A61B 3/107**, G01B 11/24

(86) Internationale Anmeldenummer:
**PCT/DE95/01579**

(87) Internationale Veröffentlichungsnummer:
**WO 96/14794 (23.05.1996 Gazette 1996/23)**

(54) **VERFAHREN ZUM BESTIMMEN DER ABSOLUTEN RAUM-KOORDINATEN MINDESTENS EINES PUNKTES EINER SPIEGELNDEN OBERFLÄCHE**

METHOD OF DETERMINING ABSOLUTE SPATIAL CO-ORDINATES OF AT LEAST ONE POINT ON A REFLECTING SURFACE

PROCEDE POUR LA DETERMINATION DES COORDONNEES SPATIALES ABSOLUES D'AU MOINS UN POINT D'UNE SURFACE REFLECHISSANTE

(84) Benannte Vertragsstaaten:
**DE FR GB IT**

(30) Priorität: **14.11.1994 DE 4440573**
**04.05.1995 DE 19516309**

(43) Veröffentlichungstag der Anmeldung:
**03.09.1997 Patentblatt 1997/36**

(73) Patentinhaber:
- **Von Wallfeld, Axel**
  **71032 Böblingen (DE)**
- **Matallana Kielmann, Michael**
  **72764 Reutlingen (DE)**
- **Oltrup, Theo**
  **72072 Tübingen (DE)**
- **Bende, Kıaus Thomas**
  **72116 Mössingen (DE)**

(72) Erfinder:
- **Von Wallfeld, Axel**
  **71032 Böblingen (DE)**

- **Matallana Kielmann, Michael**
  **72764 Reutlingen (DE)**
- **Oltrup, Theo**
  **72072 Tübingen (DE)**
- **Bende, Kıaus Thomas**
  **72116 Mössingen (DE)**

(74) Vertreter:
**Castell, Kıaus, Dr.-Ing. et al**
**Patentanwaltskanzlei**
**Liermann - Castell**
**Schillingsstrasse 335**
**52355 Düren (DE)**

(56) Entgegenhaltungen:
**EP-A- 0 397 962**          **WO-A-95/10220**
**DE-A- 4 325 494**

- **INVESTIGATIVE OPHTALMOLOGY & VISUAL SCIENCE, Bd. 25, Nr. 12, Dezember 1984 Seiten 1426-1435, S. D. KLYCE 'Computer-Assisted Corneal Topography'**

**Beschreibung**

Die Erfindung betrifft ein Verfahren zur Bestimmung der absoluten Raum-Koordinaten mindestens eines Punktes einer spiegelnden Oberfläche, bei dem ein Punkt auf der Oberfläche gespiegelt wird, der einen Abstand h zu einer optischen Achse hat und dessen Lot zu dieser optischen Achse den Punkt 0 definiert, und ein beliebiger Punkt D in einem Abstand d vom Punkt 0 auf der optischen Achse als Scheitelpunkt des Fixationswinkels $\varphi$ von $P_1$ festgelegt wird.

Verfahren zur Vermessung von spiegelnden Oberflächen sind allgemein bekannt. Sie basieren auf dein Prinzip, daß ein Punkt mit fixen räumlichen Koordinaten auf der Oberfläche gespiegelt wird und das virtuelle Bild mit einem Videokeratometer vermessen wird. Hierzu sind verschiedene Verfahren und Vorrichtungen bspw. aus dem U.S.-Patent Nr. 5,106,183, dem U.S.-Patent 5,110,200 und der deutschen Offenlegungsschrift 40 30 002 bekannt. Ein gattungsgemäßes Verfahren ist aus "Computer assisted Corneal Topography" in Investigative Ophtalmology & Visual Science, Bd. 25, Nr. 12, Dezember 1994, S. 1426 - 1435 bekannt. EP-A-397962 beschreibt ein iteratives Verfahren, in dem unbekannte Werte vom Radius $R_1$ iterativ präzisiert werden. WO-A-95/10220 beschreibt Verfahren, bei denen die iterative Methode insbesondere zur Lösung von Gleichungen mit drei Unbekannten angewendet wird.

Diese Verfahren sind dazu geeignet, relative Daten zu ermitteln. Sie eignen sich jedoch nicht dazu, einzelne Punkte einer spiegelnden Oberfläche mit ihren genauen Raum-Koordinaten zu bestimmen.

Der Erfindung liegt daher die Aufgabe zugrunde, ein Verfahren vorzustellen, mit dem einzelne Raum-Koordinaten einer spiegelnden Oberfläche bestimmbar sind. Diese Aufgabe wird mit den Merkmalen des Patentanspruchs 1 gelöst.

Das im Stand der Technik auftretende Problem der Vermessung von einzelnen Raum-Koordinaten beruht darauf, daß bei der genauen Vermessung der Maße eines virtuellen Bildes Annahmen zur Lage des virtuellen Bildes im Raum gemacht werden müssen, die nicht unbedingt genau den tatsächlichen Verhältnissen entsprechen. Eine gleichzeitige Messung der Maße des virtuellen Bildes und des Abstandes des virtuellen Bildes von einer fixen Raum-Koordinate scheidet jedoch aus mathematischen Gründen aus.

Der Erfindung liegt die Erkenntnis zugrunde, daß eine beliebige Position des virtuellen Bildes im Raum angenommen werden kann, sofern iterativ die gemessenen Werte mit den berechneten Werten solange verglichen werden, bis sie übereinstimmen. Das interative Verfahren erschließt somit eine Möglichkeit, sowohl die genauen Maße des virtuellen Bildes zu bestimmen, als auch dessen Abstand zu einem fixen Punkt.

Vorteilhaft ist es, wenn der Punkt $P_1$ ein Punkt einer Placidoscheibe ist. Durch Aufteilen einer Placidoscheibe in Segmente können beliebig viele reflektierende Punkte vermessen werden, die aufgrund ihres Ortes und vorzugsweise ihrer Farbe bei der Vermessung leicht wiedergefunden werden können.

Ein bevorzugtes Anwendungsgebiet der Erfindung ist die Vermessung der Augenhornhaut, bspw. um die Form starrer Kontaktlinsen zu bestimmen.

Um die mögliche Lage des Punktes D auf der optischen Achse einzugrenzen, ist es vorteilhaft, wenn der Abstand A zwischen dein Punkt 0 und dem Schnittpunkt S von spiegelnder Oberfläche und optischer Achse durch Einblendung mindestens eines Zentrierobjektes $P_2$ bestimmt wird.

Ein Ausführungsbeispiel der Erfindung sieht vor, daß der Abstand $l_1{}^*$ nach der Formel

$$l_1 = x \tan \alpha + h$$

mit

$$\tan \alpha = -h/(d - R_s/2)$$

und

$$x = -\frac{h \tan\alpha - 2d}{1 + \tan^2\alpha} - \sqrt{\left(\frac{h \tan\alpha - 2d}{1 + \tan^2\alpha}\right)^2 - \frac{h^2 - R_s{}^2 + d^2}{1 + \tan^2\alpha}}$$

berechnet wird.

Der Abstand $l_2$ ist vorzugsweise mit einem Videokeratometer zu messen.

Eine weitere Ausgestaltung der Erfindung sieht vor, daß durch Bestimmung der Raum-Koordinaten einer Vielzahl an Punkten und einer Intrapolation die gesamte Form einer spiegelnden Oberfläche ermittelt wird.

Ein Ausführungsbeispiel des erfindungsgemäßen Verfahrens ist in den Zeichnungen dargestellt und wird im folgenden näher beschrieben.

Es zeigt:

EP 0 792 121 B1

Figur 1    eine schematische Darstellung der geometrischen Verhältnisse und

Figur 2    ein Beispiel einer Kalibrierfunktion

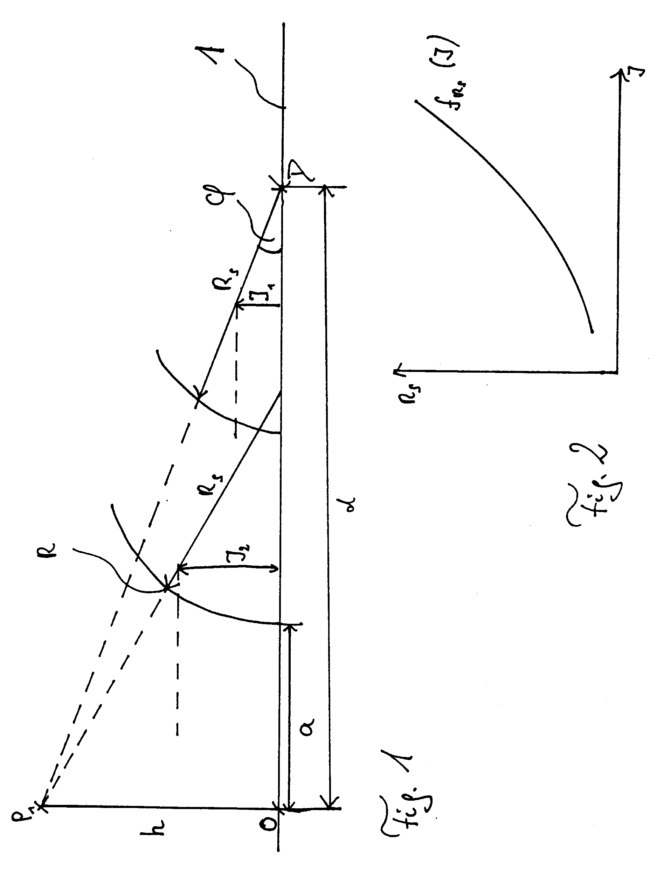

Zur Vermessung der Hornhaut eines Auges wird der spiegelnde Tränenfilm des Auges verwendet, in dem sich beliebige Punkte, wie auch der Punkt $P_1$ in Figur 1 spiegeln. Werden möglichst viele Punkte der Oberfläche ermittelt, kann durch Intrapolation die gesamte Form der spiegelnden Oberfläche der Hornhaut ermittelt werden.

Zur Ermittlung der Raumkoordinaten wird eine Placidoscheibe mit Ringen unterschiedlicher Farben auf den Tränenfilm des Auges gespiegelt und es wird zunächst ein Punkt $P_1$, der den Abstand h zu optischen Achse 1 hat, mit einem Videokeratometer auf der reflektierenden Oberfläche beobachtet.

Die optische Achse 1 weist einen Nullpunkt O auf, der sich durch Fällen eines Lotes vom Punkt $P_1$ auf die optische Achse 1 ergibt. Außerdem wird auf der optischen Achse 1 ein beliebiger Punkt D in einem Abstand d vom Nullpunkt O festgelegt, der als Fixationswinkel $\varphi$ von $P_1$ betrachtet wird.

Anschließend wird der Abstand $l_1$, der den Abstand des virtuellen Bildes von $P_1$ zu optischen Achse angibt, berechnet. Hierbei wird von der grundlegenden Beziehung

$$l_1 = x \tan \alpha + h$$

mit

$$\tan \alpha = -h/(d - R_s/2)$$

und

$$x = - \frac{h \tan\alpha - 2d}{1 + \tan^2\alpha} - \sqrt{\left(\frac{h \tan\alpha - 2d}{1 + \tan^2\alpha}\right)^2 - \frac{h^2 - R_s^2 + d^2}{1 + \tan^2\alpha}}$$

ausgegangen.

Durch das Festlegen des Punktes D auf der optischen Achse sind für diese Gleichtungsbeziehungen alle Werte bekannt. Der Wert für den Sagitalradius RS ergibt sich aus einer Kalibrierfunktion, die in Figur 2 als Beispiel dargestellt ist. Bei der Kalibrierung werden definierte Sphären mit einen Videokeratometer vermessen, um die Funktion RS = f (i) zu bestimmen.

Außerdem wird der Abstand $l_2$ des virtuellen Bildes des Punktes $P_1$ zur optischen Achse gemessen und auch bei dieser Messung wird der vorher durch Kalibrierung ermittelte Sagitalradius zugrundegelegt.

Da der Punkt D ein beliebiger Punkt auf der optischen Achse ist, ist davon auszugehen, daß der berechnete Wert $l_1$ nicht mit dem gemessenen Wert $l_2$ übereinstimmt. Falls D zu weit vom Nullpunkt O entfernt ist, ist der berechnete Abstand $l_1$ kleiner als der gemessene Abstand $l_2$ und daraufhin wird der Abstand d verringert. Mit dem dabei entstehenden neuen Punkt D wird die gleiche Berechnung wieder durchgeführt, um an Ende die Abstände $l_1$ und $l_2$ zu vergleichen. Die Annäherung des Punktes D an den Punkt D*, bei dem $l_1{}^* = l_2{}^*$ ist, kann beispielsweise durch Intervallhalbierung vorgenommen werden. Die Genauigkeit des Verfahrens wird durch die Auflösung des Videotopometers bestimmt und spätestens wenn diese Genauigkeit erreicht ist, wird das Verfahren abgebrochen, um aus dem Fixationswinkel und dem Abstand $l_1{}^* = l_2{}^*$ die Raumkoordinate des Punktes der spiegelnden Oberfläche zu berechnen. Auf diese Art und Weise können eine Vielzahl an Raumkoordinaten bestimmt werden, in denen verschiedene Punkte P auf dem Tränenfilm des Auges reflektiert werden und das Verfahren für jeden dieser Punkte durchgeführt wird. Dadurch ergibt sich ein Datensatz, der die Form der Oberfläche beschreibt. Die Bereiche zwischen den berechneten Punkten werden durch Extrapolation bestimmt.

Um den Bereich, in den, der Punkt D auf der optischen Achse 1 liegt, einzuschränken, ist es sinnvoll, zunächst den Abstand a zwischen dem Punkt O und dein Schnittpunkt S von spiegelnder Oberfläche und optischer Achse durch Einblendung mindestens eines Zentrierobjekts zu bestimmen. Hierbei ist es möglich, zwei Laserstrahlen in einen Winkel so auf die Oberfläche zu richten, daß sich ihre Reflektionspunkte überschneiden und aus dein Winkel auf die Entfernung zurückzurechnen. Außerdem ist es möglich, nur einen Laserstrahl auf die Oberfläche so zu richten, daß der Abstand des Bildes zur optischen Achse Null wird, um aus dieser geometrischen Beziehung den Abstand a zu berechnen. Dieses Verfahren ist ausführlich in der WO 94/16611 beschrieben.

Durch die Bestimmung des Punktes S auf dem Scheitelpunkt des Meßkörpers kann in Verbindung mit den nach dem erfindungsgemäßen Verfahren bestimmten Raumkoordinaten eine echte Höhenliniendarstellung durchgeführt werden. Durch die Kombination des Iterationsverfahrens und der Bestimmung des Abstands a, der sogen. z-Korrelation, läßt sich somit jeder Abstand zwischen dem System und der Oberfläche des Meßkörpers bestimmen.

Außerdem läßt sich mit diesen Daten und einer geometrischen und/oder einen quantenmechanischen Wellenanalyse (Snell'sche Gesetz) ein Strahlverfolgungsbild aufbauen. Ausgehend von einem bekannten Projektionskörper (parallele Strahlen, Gittermuster usw.) läßt sich die Abbildungsverzerrung durch die Oberfläche des Meßkörpers berechnen. Dies kann sowohl axial (bezogen auf eine optische Achse) als auch "instantanously" unter Bestimmung des echten lokalen Radius ohne jegliche Annahme oder Einschränkung durchgeführt werden.

Nach Ermittlung verschiedener Raumkoordinaten der spiegelnden Oberfläche, kann die Oberfläche durch ein Polynom n-ter-Ordnung, ein Zernike-Polynom oder eine Spline-Funktion beschrieben werden. Außerdem kann auf der Basis der ermittelten Daten die Simulation der Sagitalradien-Methode durchgeführt werden und die Daten bzw. Koeffizienten aus der simulierten Sagitalradienmethode können in einer relativen und absoluten Farbskalierung dargestellt werden.

Weiterhin wird vorgeschlagen, daß ein Kurvaturvergleich die Bestimmung von konkaven und konvexen Strukturen erlaubt und die positiven (konvexen) und negativen (konkaven) Radien in einer relativen oder absoluten Farbskalierung dargestellt werden.

**Patentansprüche**

1. Verfahren zum Bestimmen der absoluten Raum-Koordinaten mindestens eines Punktes einer spiegelnden Oberfläche

   - bei dein ein Punkt $P_1$ auf der Oberfläche gespiegelt wird, der einen Abstand h zu einer optischen Achse 1 hat und dessen Lot zu dieser optischen Achse den Punkt 0 definiert, und
   - ein beliebiger Punkt D in einem Abstand d vom Punkt 0 auf der optischen Achse als Scheitelpunkt des Fixationswinkels $\varphi$ von $P_1$ festgelegt wird, **dadurch gekennzeichnet, daß**
   - der Abstand $l_1$ des virtuellen Bildes des Punktes $P_1$ zur optischen Achse 1 mit D als Scheitelpunkt des Fixationswinkels berechnet wird,
   - der Abstand $l_2$ des virtuellen Bildes des Punktes $P_1$ zur optischen Achse 1 gemessen wird,
   - d solange variiert wird bis der gemessene Abstand $l_2{}^*$ mit dem berechneten Abstand $l_1{}^*$ bei $D^*$ übereinstimmt und
   - aus dem zugehörigen Fixationswinkel $\varphi^*$ und dem Abstand $l_1{}^*=l_2{}^*$ eine Raum-Koordinate der spiegelnden Oberfläche berechnet wird.

2. Verfahren nach Anspruch 1, **dadurch gekennzeichnet**, daß der Punkt $P_1$ ein Punkt eines Leuchtringes einer Placidoscheibe ist.

3. Verfahren nach einein der vorhergehenden Ansprüche, **dadurch gekennzeichnet**, daß die Oberfläche der spiegelnde Tränenfilm eines Auges ist.

4. Verfahren nach einem der vorhergehenden Ansprüche, **dadurch gekennzeichnet**, daß der Abstand a zwischen dem Punkt 0 und dem Schnittpunkt S von spiegelnder Oberfläche und optischer Achse durch Einblendung mindestens eines Zentrierobjektes $P_2$ bestimmt wird.

5. Verfahren nach einem der vorhergehenden Ansprüche, **dadurch gekennzeichnet**, daß der Abstand $l_1{}^*$ nach der Formel

$$l_1 = x \tan \alpha + h$$

mit

$$\tan \alpha = -h/(d-R_s/2)$$

und

$$x = -\frac{h \tan\alpha - 2d}{1 + \tan^2\alpha} - \sqrt{\left(\frac{h \tan\alpha - 2d}{1 + \tan^2\alpha}\right)^2 - \frac{h^2 - R_s{}^2 + d^2}{1 + \tan^2\alpha}}$$

4

bestimmt wird.

$l_1$   Abstand des virtuellen Bildes des Punktes $P_1$ zur optischen Achse 1
h    Abstand des Punkte $P_1$ zur optischen Achse 1
d    Abstand des Punktes D von Punkt 0
$R_s$   Sagitalradius

**6.** Verfahren nach einein der vorhergehenden Ansprüche, **dadurch gekennzeichnet**, daß der Abstand $l_2$ mit einem Videokeratometer gemessen wird.

**7.** Verfahren nach einen der vorhergehenden Ansprüche, **dadurch gekennzeichnet**, daß durch Bestimmung einer Vielzahl an Raum-Koordinaten und einer Intrapolation die Form einer spiegelnden Oberfläche ermittelt wird.

**Claims**

**1.** A method of determining the absolute spatial coordinates of at least one point on a reflecting surface,

- whereby a point $P_1$ on the surface is reflected, [the point] being a distance h from an optical axis 1, and its plumb line to this optical axis defines point 0, and
- any point D at a distance d from point 0 on the optical axis is defined as the vertex of the fixation angle $\varphi$ of $P_1$, characterized in that
- the distance $l_1$ of the virtual image of point $P_1$ from optical axis 1 is calculated with D as the vertex of the fixation angle,
- the distance $l_2$ of the virtual image of point $P_1$ from optical axis 1 is measured,
- d is varied until the measured distance $l_2^*$ matches the calculated distance $l_1^*$ at $D^*$, and
- a spatial coordinate of the reflective surface is calculated from the respective fixation angle $\varphi^*$ and the distance $l_1^* = l_2^*$.

**2.** A method according to claim 1, characterized in that the point $P_1$ is a point on a light ring of a keratoscope.

**3.** A method according to one of the preceding claims, characterized in that the surface is the reflective lacrimal film of the eye.

**4.** A method according to one of the preceding claims, characterized in that the distance a between point 0 and the point of intersection S of the reflective surface and the optical axis is determined by the overlay of at least one centering object $P_2$.

**5.** A method according to one of the preceding claims, characterized in that distance $l_1^*$ is determined according to the formula:

$$l_1 = x \tan \alpha + h$$

where

$$\tan \alpha = -h/(d - R_s/2)$$

and

$$x = -\frac{h \tan\alpha - 2d}{1 + \tan^2\alpha} - \sqrt{\left(\frac{h \tan\alpha - 2d}{1 + \tan^2\alpha}\right)^2 - \frac{h^2 - R_s^2 + d^2}{1 + \tan^2\alpha}}$$

$l_1$   Distance between the virtual image of point $P_1$ and optical axis 1
h    Distance between point $P_1$ and optical axis 1
d    Distance between point D and point 0
$R_s$   Sagittal radius

6. A method according to one of the preceding claims, characterized in that the distance $l_2$ is measured with a video keratometer.

7. A method according to one of the preceding claims, characterized in that the shape of a reflective surface is ascertained by determination of several spatial coordinates and interpolation.

**Revendications**

1. Procédé pour la détermination des coordonnées spatiales absolues d'au moins un point d'une surface réfléchissante,

   - selon lequel on provoque la réflexion d'un point $P_1$ sur la surface, point présentant une distance h par rapport à un axe optique 1 et dont la perpendiculaire à cet axe optique définit le point 0, et
   - on localise un point quelconque D à une distance d du point O sur l'axe optique, en tant qu'apex de l'angle de fixation φ de $P_1$, caractérisé en ce que :
   - on calcule la distance $l_1$ de l'image virtuelle du point $P_1$ par rapport à l'axe optique 1 avec D en tant qu'apex de l'angle de fixation,
   - on mesure la distance $l_2$ de l'image virtuelle du point $P_1$ par rapport à l'axe optique 1,
   - on fait varier d jusqu'à ce que la distance mesurée $l_2{}^*$ coïncide avec la distance $l_1{}^*$ calculée dans le cas de D*, et
   - on calcule une coordonnée spatiale de la surface réfléchissante sur la base de l'angle de fixation φ* correspondant et de la distance $l_1{}^* = l_2{}^*$.

2. Procédé selon la revendication 1, caractérisé en ce que le point $P_1$ est un point d'un anneau lumineux d'un kératoscope.

3. Procédé selon l'une des revendications qui précèdent, caractérisé en ce que la surface est le film lacrymal d'un oeil.

4. Procédé selon l'une des revendications qui précèdent, caractérisé en ce qu'on détermine la distance a comprise entre le point O et le point d'intersection S de la surface réfléchissante et de l'axe optique en faisant apparaître en fondu au moins un objet à centrer $P_2$.

5. Procédé selon l'une des revendications qui précèdent, caractérisé en ce que la distance $l_1{}^*$ est déterminée suivant la formule

$$l_1 = x \tan \alpha + h$$

avec

$$\tan \alpha = -h/(d - R_s/2)$$

et

$$x = -\frac{h \tan\alpha - 2d}{1 + \tan^2\alpha} - \sqrt{\left(\frac{h \tan\alpha - 2d}{1 + \tan^2\alpha}\right)^2 - \frac{h^2 - R_s{}^2 + d^2}{1 + \tan^2\alpha}}$$

$l_1$    Distance de l'image virtuelle du point $P_1$ par rapport à l'axe optique 1
h    Distance du point $P_1$ par rapport à l'axe optique 1
d    Distance du point D au point O
$R_s$    Rayon sagittal

6. Procédé selon l'une des revendications qui précèdent, caractérisé en ce que la distance $l_2$ est mesurée avec un kératomètre vidéo.

7. Procédé selon l'une des revendications qui précèdent, caractérisé en ce qu'on détermine la forme d'une surface réfléchissante par détermination de plusieurs coordonnées spatiales et d'une interpolation.

fig. 1

fig. 2